# EUROPEAN PATENT APPLICATION

(11) **EP 4 740 840 A1**
(43) Date of publication of application: **13.05.2026**
(21) Application number: 24212978.1
(22) Date of filing: 14.11.2024
(51) Int. Cl.: A61B 5/00

(54) **ELASTOMER SUPPORT ASSEMBLY FOR USE IN CONNECTION WITH A WEARABLE DEVICE**

(30) Priority: 06.11.2024 US 202418938518
(71) Applicant: BackAware Belt Ltd., Kilkenny R95 ARR7 (IE)
(72) Inventor: Everard, Eoin, Kilkenny, R95 ARR7 (IE)
(74) Representative: Weisse, Moltmann & Willems PartGmbB

(57) **Abstract**

The application relates to an elastomer support assembly designed for integration with wearable devices to monitor back posture, particularly spinal position in real-time. The assembly includes an elastomer support with a circumferential groove, dividing it into two sections for secure attachment to a wearable device, such as a belt or garment. A detachable sensor within the elastomer support captures lower back movements. The assembly ensures consistent contact with the user's lumbar spine, even during dynamic activities, enhancing data accuracy. An electronic control unit processes sensor data, detects deviations from ergonomic thresholds, and provides feedback, such as haptic alerts.

## Description

The present application relates to a back posture monitoring device and more particularly relates to an elastomer support assembly for use in connection with a wearable device for detecting lower back movement of a user.

### BACKGROUND

It is well known that improper posture and exercise technique leads to muscular fatigue or more serious defects including disc herniations or repetitive stress injuries (RSI). Poor exercise technique and posture can have a detrimental effect on back health leading to issues such as bulging discs or low back pain.

Devices which are designed to detect poor back posture and alert a user when such posture is detected are known in the art. Typically, such devices comprise a garment or belt, fitted with sensors that collect data on the back's parameters to indicate and monitor posture.

A poor back posture or poor position of the spine, as disclosed herein, refers preferably to any deviation from its natural, ergonomic alignment, which may include excessive slouching, overextension, flexion, or misalignment that places undue stress on the spinal column. These poor positions can vary depending on the activity or situation. For instance, a poor spinal position while standing may differ significantly from one while sitting, performing squats, lifting weights, doing sit-ups, or practicing yoga.

### PRIOR ART

Existing systems such as sensors embedded in garments or belts may not conform to the natural curvature of the lower back, specifically the lordotic region, leading to inaccurate detection of user's low back position or lumbar spine position. Such misalignment may result in inconsistent feedback, thereby reducing the reliability of the data gathered during motion in the lower back either when sitting, lifting or exercising.

Existing systems often compensate for poor sensor attachment by increasing the sensitivity of the sensors to ensure some level of accuracy despite an inability to replicate the subtle movements of the lumbar spine. However, this increased sensitivity can result in false positives, where normal movements or slight shifts are interpreted as significant spinal misalignments. This issue exacerbates the unreliability of these systems, while providing users with incorrect feedback and potentially leading to unnecessary interventions or adjustments in posture when none are required. On the other hand, if the sensor in the garment does not stay connected to the lower back (Lumbar Spine), then subtle but important movements of the lower back may be missed. Therefore, it is necessary that the sensors stay in congruency with the lower back at all times and replicates the exact movements of the low back, however subtle.

Belts and garments are often designed with one-size-fits-all solutions that do not account for individual anatomical differences, particularly in the lower back's lordotic curvature. Without a system that naturally adjusts to these variations, users with different body types may experience discomfort and ineffective sensor performance, as the sensor may not remain tight against the skin, further compromising data accuracy.

Furthermore, a critical issue in existing systems is the risk of sensor detachment during motion. Belts and garments may become loose or shift position, especially during activities involving bending, twisting, or dynamic movement.

An example of a back monitoring device is disclosed in US 2019/0333407 A1, which describes a system for providing tactile input for prompting a person to coordinate appropriate muscle contractions during some form of physical movement such as exercising. The system may comprise a belt bearing inwardly facing projections adapted to make contact with a user's skin. The user may then be prompted by sensory tactile feedback or by audible, visible, or vibratory outputs to facilitate proper contractions, timing, and sequencing of activities, as may be appropriate given the type of physical activity. In one embodiment, the system may include paraspinal muscle sensors that make contact with the paraspinal muscles on either side of the spine when the belt is worn. These sensors provide feedback regarding the stabilizing function and movement of the back.

However, the disadvantage of this system is that it only captures muscle parameters and does not provide direct and precise feedback on the posture of the user's spine.

### OBJECT OF THE INVENTION

The object of the invention was therefore to overcome the disadvantages of the prior art and to provide a back posture monitoring device that delivers direct data on spinal posture and in particular a reliable and improved sensor assembly integrated into a wearable device to ensure consistent positioning on the user's spine.

### DISCLOSURE OF THE INVENTION

The object of the invention is solved by the features of the independent claims. Advantageous embodiments of the invention are described in the dependent claims.

In a first aspect, the application relates to an elastomer support assembly for use in connection with a wearable device to monitor back posture, in particular real time spinal position. The elastomer support assembly comprises an elastomer support and a sensor detachably integrated within the elastomer support. The elastomer support is in this respect attachable to the wearable device and adhering directly to the lumbar spine of a user when the wearable device is worn by the user for allowing sensors to detect lower back movement of the user.

This design is unique in comparison to prior art systems, as the sensor assembly is positioned directly against the spine of the user when it is worn by a user in conjunction with a wearable device, enabling the direct and immediate recording of spinal posture. The attachment method ensures that the sensor assembly remains in place during user movement. Additionally, because the elastomer support assembly is attachable and detachable, it can function as a standalone component that can be easily paired with various wearable devices. This versatility allows the system to be adapted for users of different sizes who may require different wearable configurations. The removable design also facilitates easy maintenance and sensor replacement. Furthermore, the sensors are strategically arranged and encapsulated in a material that optimizes the transmission of spinal posture and movement data.

According to the application an elastomer support is preferably a flexible, stretchable structure made from elastomer materials that can deform and return to its original shape. It is designed to provide a secure and adaptable fit against a surface, such as the human body, maintaining consistent contact even during movement. In wearable devices, an elastomer support ensures that sensors or components stay aligned with the body's contours, especially in areas like the lower back, to enhance the accuracy of data collection and user comfort. Preferably, the elastomer support is made of foam, rubber and/or silicone. The flexibility of these materials ensures that the support can conform to the user's specific body contours, thereby being suitable for a wide range of body types and postures. Silicone and rubber, in particular, are resistant to moisture, making the support ideal for use during exercise or in humid environments, as it prevents sensor malfunction or damage from sweat. The material rubber may comprise a thermoplastic elastomer as for example SEBS (Styrene-EthyleneButylene-Styrene) or a natural latex rubber. An example of a material that could represent foam is polyurethane foam (PU foam).

The foam may comprise a closed-cell structure; however, an open-cell foam is more preferred. Open-cell foam generally provides a softer, more flexible support surface, which enhances pressure distribution and minimizes the risk of pressure points, thereby improving user comfort during extended periods of sitting or use.

As described, the sensor is incorporated into the elastomer support in a way that allows it to be easily attached and removed. The sensor is securely held within the flexible structure but can be taken out or replaced as needed without damaging the support.

In a preferred embodiment the elastomer support comprises a circumferential groove, which divides the elastomer support into a first and second support section. In this regard, the elastomer support is attachable to the wearable device through an opening that matches the groove, positioning the first support section on an inner side and the second support section on an outer side of the wearable device with the first support section adhering to the lumbar spine of a user when the wearable device is worn by the user. This configuration provides a secure yet easily detachable attachment to the wearable device, ensuring that the support assembly remains correctly positioned for optimal sensor performance. Additionally, it allows for easy removal for maintenance or replacement in the event of damage or wear.

The inner side of the wearable device preferably refers to the side of the wearable device that faces and comes into direct contact with the user's body when the device is worn. In this context, the first support section of the elastomer support is positioned on this side to adhere directly to the lumbar spine.

On the other hand, the outer side of the of the wearable device preferably refers to the side of the wearable device that faces away from the user's body and is exposed to the external environment. The second support section of the elastomer support is positioned on this side to provide structural stability and maintain the attachment to the device.

Moreover, the outer contour of the second support section may have smaller dimensions than that of the first section. The smaller dimensions of the outer contour of the second support section reduce the bulkiness of the wearable device with the elastomer support assembly, allowing it to protrude less from the outer side and enabling the user to comfortably wear additional clothing, such as a sweater or shirt, over it. In contrast, the first section is made larger to house the sensor electronics and ensure a broader contact area with the user's back, which improves data acquisition and accuracy.

In other words, the depth, height, and/or width of the second support section can be smaller than the depth, height, and/or width of the first support section.

Together, both sections of the elastomer support can form a compact rectangular shape with rounded corners. In this regard, the height of the elastomer support can be greater than its width and depth. When attached to the wearable device, the elastomer support is preferably oriented so that its greatest dimension, such as its height, aligns with the user's spine to maximize surface contact. The width of the elastomer support roughly corresponds to the width of the spine, as there is no need to monitor the surrounding muscles with the sensors.

Furthermore, the width of the groove of the elastomer support substantially corresponds to the thickness of the wearable device. The width of the groove is preferably defined by the measurement of the distance between the inner and outer edges of the groove in the elastomer support. It preferably defines how wide the groove is and is designed to match the thickness of the wearable device, ensuring a secure and stable fit when the support is attached

As mentioned above, the sensor may be detachably integrated within the elastomer support. In this regard, the first support section of the elastomer support may comprise a cavity in which the sensor is placed. The cavity is preferably accessible from the outside, allowing the sensor to be connected to a wire and allowing the sensor to be removed from the elastomer support.

The term "outside" preferably refers to the external surface of the elastomer support that is exposed to the environment. In the context of the cavity within the first support section, "outside" indicates the accessible opening of the cavity through which the sensor can be inserted, removed, or connected to external components, such as a wire.

The cavity can have various shape configurations. In particular, its shape is designed to securely position the sensor within the cavity and to ensure that changes in spinal position or forces effectively transfer through the elastomer support material to the sensor. Preferably, the sensor makes contact with the walls of the cavity and/or can be securely clamped within it.

The sensor may include a position detection sensor. The inclusion of a position detection enables to precisely track spinal alignment and subtle movements.

Moreover, the sensor may comprise a flex sensor, a strain sensor, and any other position and/or displacement detection sensors. The integration of a flex sensor, strain sensor, or other position and displacement detection sensors provides the ability to measure dynamic changes in spinal curvature and movement with high precision. These sensors enhance the device's capability to detect subtle deformations and strains in the lower back.

In a preferred embodiment, the flex sensor, strain sensor, and any other position and/or displacement detection sensors are mounted on a sensor carrier, and the sensor carrier is located in the cavity of the first support section of the elastomer support.

The sensor carrier serves in particular as a stable platform for securely mounting the flex sensor, strain sensor, and other position and/or displacement detection sensors within the cavity of the first support section. By attaching the sensors on a dedicated carrier, it ensures their proper alignment and optimal positioning for accurate data collection. Additionally, the sensor carrier facilitates easy installation and removal of the sensors. Its design may include features such as grooves, slots, or clamps to hold the sensors firmly in place, preventing movement or dislodgment during dynamic activities. The carrier can also provide protection to the sensors by shielding them from mechanical stress, moisture, or other external factors. The sensor carrier may be designed as a flat, elongated rod-like element.

In a further preferred embodiment, the elastomer support assembly comprises an electronic control unit which is connected to the sensor or sensors via a wire to continuously transmit sensor data related to back posture of the user in real-time. The wires are preferably conductive connectors used to transmit electrical signals between the sensors and the control unit within the elastomer support assembly. The wires can be understood as cables.

The control unit is preferably configured to process and analyse the sensor data. In this respect, the sensor data may include spine position data of the user and the control unit is preferably configured to detect if the spine position of the user exceeds predefined thresholds.

In some embodiments, the control unit employs algorithms to determine the poor position of the user's spine by receiving the spine position data. For example, the control unit may use dimensionality reduction techniques (e.g., Principal Component Analysis (PCA), t-SNE, or autoencoders) to transform the high-dimensional sensor data into a lower-dimensional space. This transformation helps capture the essential features of the spinal position while reducing noise and computational complexity. The reduced-dimensional data can then be combined to create a simplified yet comprehensive representation of the current spinal position. The control unit may evaluate whether the current spinal position exceeds predefined thresholds. If the deviation exceeds these thresholds, a control signal may be generated and sent to activate a vibration actuator for providing immediate haptic feedback to the user.

The control unit also may include an wireless communication module to communicate sensor data to a mobile device having a mobile application for real time visualization of user's back posture using a graphical user interface (GUI) of the mobile application.

Spine position data refers preferably to measurable information captured by the sensors in the elastomer support assembly about the alignment, curvature, and movement of the user's lower back. This data preferably may distinguish between static postures and dynamic movements. The electronic control unit processes this data in real-time to detect deviations from predefined thresholds and provides feedback to help the user maintain optimal posture.

Predefined thresholds are preferably specific, preset limits or values that indicate the acceptable range of spinal position or movement for the user. These thresholds are preferably determined based on ergonomic, medical, or biomechanical standards to identify when the user's posture deviates from an optimal or safe alignment. If the spine's position or movement exceeds these thresholds, the system generates feedback or alerts to prompt corrective action. Accordingly, the predefined threshold preferably includes initially calibrated spine positions of the user.

In this context, the control unit may be in data communication with an external device, such as a mobile device or smartphone or a dedicated monitoring device, to transmit real-time feedback and posture data. This communication allows users to receive detailed analytics, alerts, and suggestions for posture improvement through an app or interface on the external device. Additionally, the control unit can be connected to vibration actuators integrated within the wearable device, which can provide immediate tactile feedback to the user when poor posture is detected. The vibration actuators are triggered when the control unit identifies deviations from predefined thresholds.

The initial calibration of spine position may also be performed using a software application in an external device.

In some embodiments, the mobile device is referred to as a smart device. The smart device includes an electronic device that is typically connected to other devices or networks via various wireless protocols such as Bluetooth, Wi-Fi, or cellular networks. The smart device can operate interactively and autonomously. In the context of the posture monitoring system, the smart device includes a smartphone, laptop or tablet that may run a mobile application which is designed to interface with the monitoring device.

Using the GUI of the mobile application, the user is able to visualize the back posture. The GUI may display the back posture through a digital sensor ball and digital flexion and extension barriers. The flexion and extension barriers are upper and lower thresholds respectively alerting the user of excessive movement. The graphical representation of the thresholds and the current posture of the spinal position allows the user to intuitively understand their posture and maintain the correct spinal position with ease.

The mobile application may include a calibration module for calibrating or recalibrating the sensor based on desired position of the user. The flexion and extension barriers return to predetermined distances from the desired position upon calibration or recalibration.

The control unit may comprise a processor, a memory, a rechargeable battery and/or a data communication unit housed in a housing attachable via attaching means to the wearable device. The housing is preferably a protective enclosure designed to securely contain and shield the components of the control unit. The housing may comprise a slot for a plug connection to connect a wire from the sensor with the control unit and/or to charge the battery.

The attaching means of the housing may comprise a hook, a button, a snap fastener or a Velcro strip.

A plug connection preferably refers to a detachable interface where one component, such as a cable or sensor, is securely connected to another component, typically through a plug-and-socket mechanism. This type of connection enables easy assembly, disassembly, and replacement of parts while ensuring reliable transmission of electrical signals or data.

Further, the housing may be is at least partially encased in a silicone cover to provide additional protection against external factors such as moisture, dust, and impacts. This silicone cover also enhances user comfort by creating a smooth, non-slip surface that minimizes irritation when in contact with the skin or clothing.

In a further preferred embodiment, the electronic control unit may be located within the elastomer support, for example, in the same cavity as the sensor or at another position within the elastomer support. It is understood that the cavity must be sufficiently large to accommodate both the sensors and the control unit.

In this context, the control unit may include a housing that encases the processor, memory, rechargeable battery, and/or data communication unit. The housing can either be integrated into the elastomer support, or the control unit, along with its components, may be directly embedded into the elastomer support. For instance, a circuit board containing the components of the control unit could be incorporated directly into the elastomer support. In particular, the wire connecting the control unit and the sensor may also be embedded within the elastomer support.

Preferably, the electronic control unit may be positioned at the center of the elastomer support. In other embodiments, the control unit may be located specifically in the first or second support section of the elastomer support. Particular preferred, the elastomer support is configured to replicate the position of the lower back of the user based on the change in user's position. The lower back movement of a user may include spine movement, low back movement, lumbar spine movement or various lordotic positions. In this context, the elastomer support is preferably flexible to conform to different body shapes without deformation such that the sensor accurately detects and responds to movements of the user.

In a further aspect, the application relates to a wearable device for use in connection with an elastomer support assembly for monitoring back posture, in particular real time spinal position, wherein the wearable device comprises an opening that matches a groove of the elastomer support of the elastomer support assembly. The wearable device serves as the counterpart to the elastomer support assembly, with both components working together to enable the user to receive accurate and actionable feedback about their back position. This interdependent design ensures that the elastomer support assembly remains securely in place, maintaining consistent contact with the user's lower back, while the wearable device provides the necessary structure and stability for effective data collection and feedback delivery.

The wearable device may comprise a belt, a garment or any other body-wearable apparatus. Belt or garment type wearables provide flexibility in how the device can be worn, enhance user comfort and ensure consistent usage over extended periods. Other possible body-wearable apparatuses include a vest, which for example houses sensing medium across the back and shoulders for comprehensive posture monitoring; a brace, providing for example additional support and ensuring sensor placement; adjustable straps worn for example over clothing; a harness for example to secure sensor placement during high-intensity activities; specially designed underwear for example for discreet monitoring; adhesive patches applied directly to the skin; a lightweight armature or exoskeleton; a specially designed backpack with integrated sensors; and arm or leg sleeves that incorporate sensors to monitor posture and related movements. Aforementioned body-wearable apparatuses enhance the flexibility and usability of the posture monitoring device, while catering to different preferences and activity levels.

An opening, in the context of the wearable device, is a specifically designed aperture or cutout that may correspond to the groove of the elastomer support. The wearable device is preferably made of a flexible or elastic material, allowing the elastomer support of the elastomer support assembly to be inserted into the opening, even if the dimensions of the elastomer support outside the groove are slightly larger than those of the opening.

The edge areas of the opening can be reinforced either with reinforcement materials or through processing techniques such as heat sealing, stitching, or adhesive bonding.

The edge areas may refer to the boundary regions or a frame immediately surrounding the opening in the wearable device, typically extending a few millimeters to a few centimeters from the perimeter of the opening, depending on the wearable device's size and material. For instance, the edge area might extend 5 mm outward from the opening and be reinforced with a 0.5 mm thick fabric overlay or a silicone strip to enhance durability and prevent wear. Additionally, these edge areas can be processed using techniques such as heat sealing, where a 3 mm wide fused boundary is created to prevent fraying or tearing. Alternatively, stitching could be applied with a double-stitched seam and a thread spacing of 2 mm to provide strength and flexibility. These reinforcements and processing techniques ensure the opening remains durable and retains its shape during repeated use.

Reinforcement materials are preferably additional substances or layers added to strengthen the edge areas of an opening in a wearable device. Examples include durable fabrics like nylon or polyester, rubberized coatings, silicone, or thermoplastic elastomers (TPE), all of which enhance resistance to tearing, stretching, or deformation during repeated use.

In a further preferred embodiment, the wearable device, designed as a belt, features different sections along its length that vary in material composition, thickness, and/or width to optimize functionality and user comfort. Preferably, a central section of the belt is positioned at the spine when worn by the user and includes the opening. The central section may have a greater height than the outer sections of the belt, and the outer sections include closure elements. The increased height of the central section provides additional stability and support to the spine. This measure enhances the effectiveness of the posture monitoring system.

The inclusion of closure elements in the outer sections facilitates easy and secure fastening, The closure elements may comprise components for a Velcro connection, a button connection, a hook-and-eye connection, a loop-and-opening connection, and/or a buckle connection.

In a further aspect the application relates to a system to monitor back posture, in particular real time spinal position. The system comprises an elastomer support assembly according to any of the embodiments above and a wearable device according to any of the embodiments above.

In this regard, the elastomer support of the elastomer support assembly is attached to the wearable device through the opening of the wearable device. The first support section is positioned on an inner side and the second support section on an outer side of the wearable device with the first support section adhering to the lumbar spine of a user when the wearable device is worn by the user for allowing sensors to detect lower back movement of the user.

Compared to the sensor-based systems for monitoring back posture known from the prior art, the proposed system addresses key challenges such as inaccurate readings, false positives, and inconvenient application, all of which undermine the reliability and practicality of existing systems in real-world scenarios.

The dynamic nature of the system ensures that the system can be worn during various exercises or daily activities without requiring the user to adjust or alter the system's arrangement. This adaptability improves the system's usability and effectiveness, as it seamlessly integrates into the user's routine while consistently providing accurate posture

The elastomer support assembly may include a control unit enclosed in a housing with a hook, the control unit being attached to the wearable device via the hook and connected to the sensor of the elastomer support assembly through a wire. The inclusion of a hook allows the control unit to be securely yet easily attached and detached from the wearable device. The wired connection ensures a stable and reliable transmission of data between the control unit and the sensor. Additionally, enclosing the control unit in a protective housing shields it from external factors such as moisture, dust, and impact.

The control unit may include or be connected to a vibration actuator to alert the user upon detecting the change in the back posture of the user. In one embodiment, the control unit may transmits the sensor data received from the posture sensor to the vibration actuator, for example as a control signal, when the control unit detects a bad or poor position of the user's spine. Upon receiving the control signal, the vibration actuator vibrates to alert the user for correcting the posture. The vibration actuator is preferably a component or a device that generates vibrations to provide haptic feedback to the user. Examples of vibration actuators include Eccentric Rotating Mass (ERM) motors, which use an off-center mass to create vibrations; Linear Resonant Actuators (LRA), which utilize a moving mass and electromagnetic coil for precise feedback; Piezoelectric actuators, which generate vibrations through the deformation of piezoelectric materials when an electric field is applied; and Voice Coil Actuators, which operate through the interaction of a magnetic field and electric current.

The skilled person will recognize that the advantages, technical effects and preferred embodiments discussed in connection with the elastomer support assembly and the wearable device apply analogously to the system to monitor back posture. Likewise, all the advantages, technical effects and preferred embodiments described in connection with the system are transferable to the elastomer support assembly and the wearable device.

Further examples of embodiments are explained in more detail below with reference to the accompanying drawings. The application is not intended to be limited solely to these listed examples of embodiments. They merely serve to explain the application in more detail. The present application is intended to relate to all objects which the person skilled in the art would use now and, in the future, as obvious to realize the invention.

### BRIEF DESCRIPTION OF THE FIGURES

- **Fig. 1**: shows a preferred embodiment of the back posture monitoring system from behind and indicates the position in which it can be attached to the user.
- **Fig. 2**: shows a preferred embodiment of the back posture monitoring system in side view and indicates the position in which it can be attached to the user.
- **Fig. 3**: shows a preferred embodiment of the back posture monitoring system in side view and indicates the forces acting on the elastomer support.
- **Fig. 4**: shows a side view of a preferred embodiment of the elastomer support.
- **Fig. 5**: shows a front view and an angled view of a preferred embodiment of the elastomeric support.
- **Fig. 6**: shows a preferred embodiment of the back posture monitoring system from behind and indicates how the elastomeric support is integrated into the wearable device.
- **Fig. 7**: shows a preferred embodiment of the back posture monitoring system from the front and shows how the elastomeric support assembly is integrated into the wearable device.
- **Fig. 8**: shows a preferred embodiment of an elastomer support assembly in a disassembled view.
- **Fig. 9**: shows a preferred embodiment Fig. 8 in an assembled view.
- **Fig. 10**: shows a partial view of a preferred embodiment of the back posture monitoring system from behind, focusing on a central section of the wearable device.
- **Fig. 11**: shows a preferred embodiment of the back posture monitoring system from behind.
- **Fig. 12**: shows a partial view of a preferred embodiment of the wearable device, focusing on an outer section of the wearable device.
- **Fig. 13**: shows different views of a silicone housing of a elastomer support assembly.
- **Fig. 14**: shows a user standing straight, wearing a belt integrated with elastomer support assembly, and a screen of the user's smart device is shown.

### DETAILED DESCRIPTION OF THE FIGURES

Some parts of the embodiments have similar or identical parts. The similar or identical parts may have the same names and/or reference number. The description of one part applies by reference to another similar part, where appropriate, thereby reducing repetition of text without limiting the disclosure.

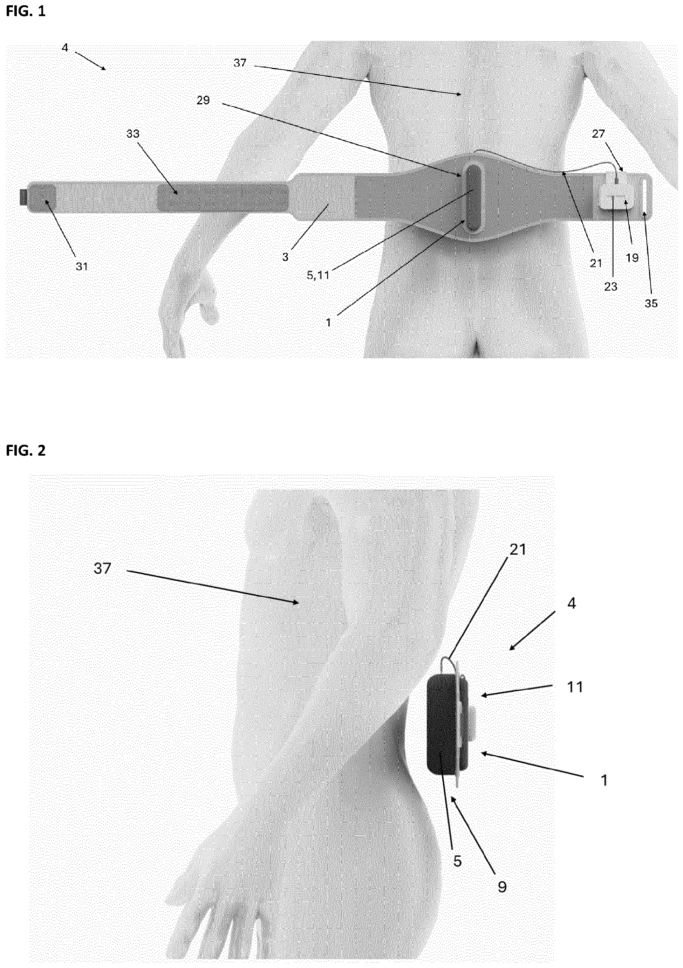

**Fig. 1** shows a preferred embodiment of the back posture monitoring system 4 from behind and indicates the position in which it can be attached to the user 37. The system 4 is specifically designed to monitor the spinal position of the user 37 in real time.

In this regard, it comprises an elastomer support assembly 1 and a wearable device 3, particularly a belt. The elastomer support assembly 1 comprises an elastomer support 5 and a sensor 13, which is detachably integrated within the elastomer support 5. The elastomer support 5 is attached to the wearable device 3 and adheres directly to the lumbar spine of the user 37 when the wearable device 3 is worn by the user 37, allowing the sensor 13 to detect lower back movement of the user 37.

The elastomer support assembly 1 further comprises an electronic control unit 19, which is connected to the sensor 13 via a wire 21 to continuously transmit sensor data related to the back posture of the user 37 in real time. The control unit 19 includes a processor, a memory, a rechargeable battery, and/or a data communication unit, all housed in a housing 23 that is attached to the wearable device 3, or belt, via a hook 27.

The sensor 13 may include a flex sensor, a strain sensor, and any other position and displacement detection sensor.

The belt comprises elastic material and features different sections along its length that vary in width or height. A central section of the belt, positioned at the spine when worn by the user 37, includes an opening 15 with reinforcement material 29, where the elastomer support 5 of the elastomer support assembly 1 is integrated. The central section has a greater height than the outer sections of the belt, with the outer sections including closure elements.

The closure elements are designed as follows: On one outer section of the belt, two velcro patches 31, 33 are arranged with a gap between them, while an closure opening 35 is positioned on the other outer section of the belt. The outer section with the velcro patches 31, 33 can be passed through the opening 35 and folded over, allowing the velcro patches 31, 33 to be placed on top of each other and connected when the user 37 wears the back posture monitoring system 4.

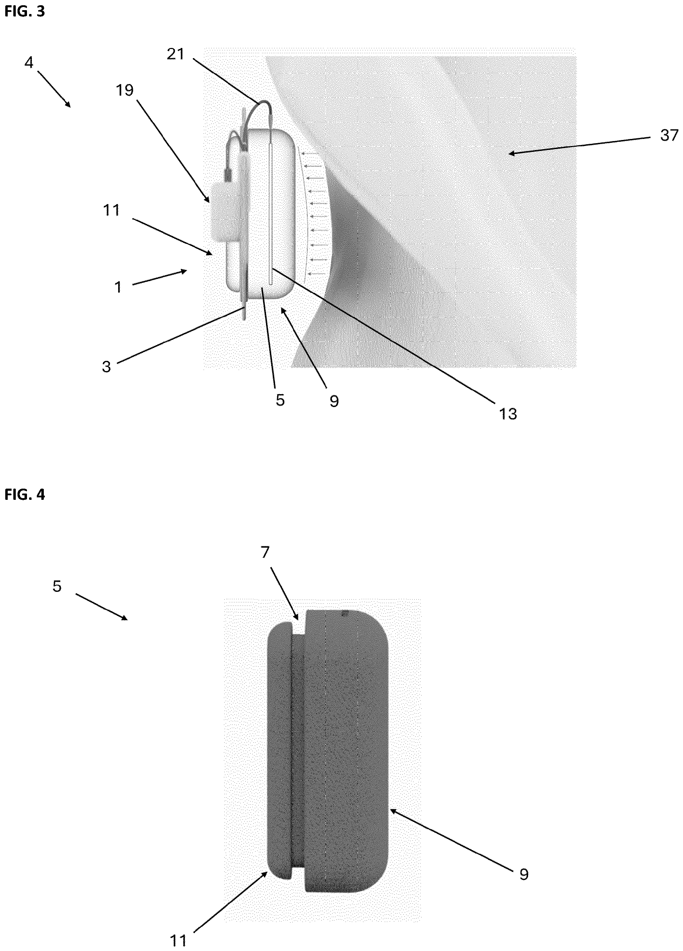

**Fig. 2** and **Fig. 3** shows a preferred embodiment of the back posture monitoring system 4 in side view and indicates the position in which it can be attached to the user 37. **Fig. 2** and **Fig. 3** correspond to the description of **Fig. 1****,** to which reference is made.

In particular, it can be seen in **Fig. 3** that a force or movement from the spine acts on the elastomer support 5 of the elastomer support assembly 1. This force or movement acts along a vertical axis, which is why the height of the elastomer support 5 is greater than its width and depth, and it is aligned with its height along the spine.

The elastomer support 5 replicates the position and movement of the lower back of the user 37 based on changes in the user's position. Accordingly, the elastomer support 5 is made of flexible materials, including foam, rubber, and/or silicone. The foam may consist of open-cell foam. The lower back movement includes spine movement, lower back movement, lumbar spine movement, or various lordotic positions. Consequently, the elastomer support 5 is flexible enough to conform to different body shapes, ensuring that the sensor 13 accurately detects and responds to the movements of the user 37.

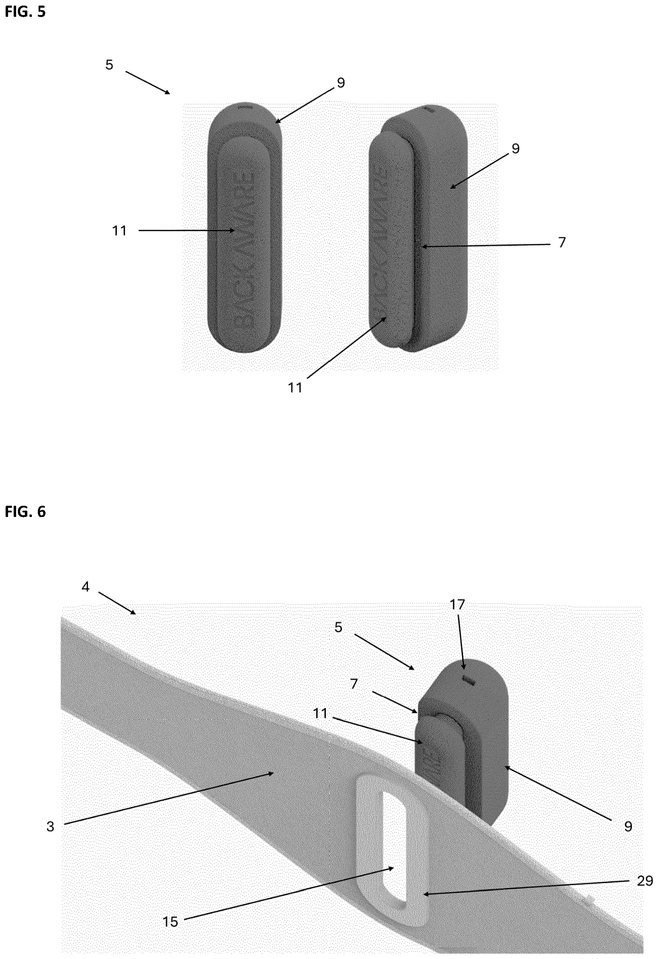

**Fig. 4** and **Fig. 5** show a preferred embodiment of the elastomer support 5 of the elastomer support assembly 1 from different views. **Fig. 4** and **Fig. 5** correspond to the description of **Fig. 1, Fig. 2** and **Fig.3****,** to which reference is made.

The elastomer support 5 includes a circumferential groove 7, which divides the elastomer support 5 into a first support section 9 and a second support section 11. The elastomer support 5 can be attached to the wearable device 3 through an opening 15 that aligns with the groove 7, positioning the first support section 9 on the inner side and the second support section 11 on the outer side of the wearable device 3. When the wearable device 3 is worn by the user 37, the first support section 9 adheres to the user's lumbar spine.

The outer contour of the second support section 11 has smaller dimensions than that of the first support section 9. Accordingly, the depth, height, and width of the second support section 11 are particularly smaller than the depth, height, and width of the first support section 9. The width of the groove 7, in turn, substantially corresponds to the thickness of the wearable device 3, ensuring that the elastomer support can be properly attached at that location.

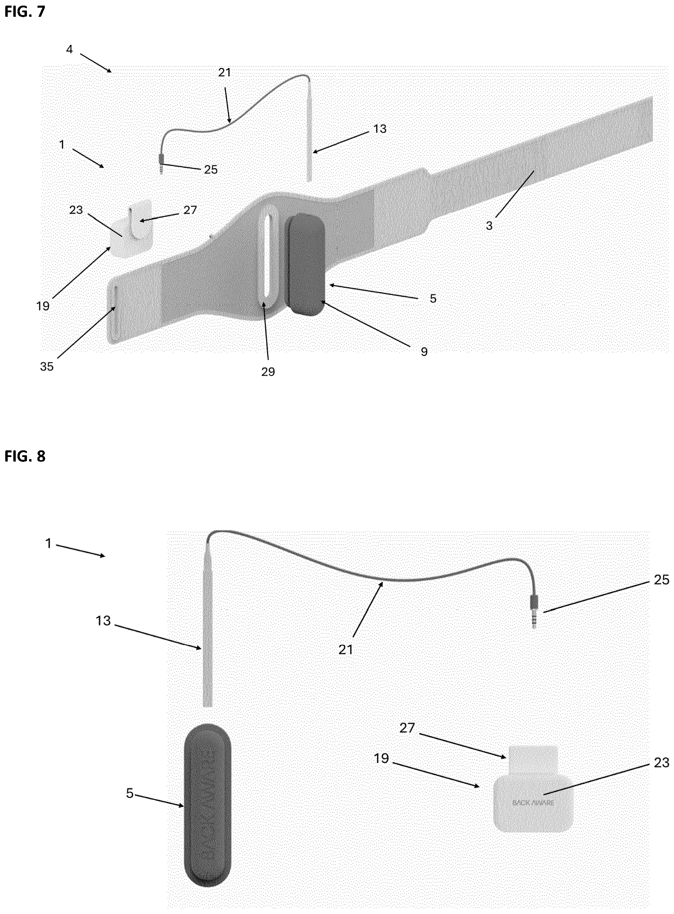

**Fig. 6** and **Fig. 7** show a preferred embodiment of the back posture monitoring system 4 from different views and illustrate how the elastomer support 5 is integrated into the wearable device. Reference is made to the descriptions of the other figures.

The first support section 9 includes, in particular, a cavity 17 designed to hold the sensor 13. The cavity 17 is accessible from the outside, enabling the sensor 13 to be connected to a wire 21 and allowing for easy insertion and removal of the sensor from the elastomer support 5.

The sensor 13 may include a variety of sensors, such as a flex sensor, strain sensor, and other position or displacement detection sensors. In this case, the flex sensor, strain sensor, and any other position or displacement detection sensors are mounted on a sensor carrier, which can be positioned within the cavity 17 of the first section 9 of the elastomer support 5.

The control unit 19 includes a processor, a memory, a rechargeable battery, and/or a data communication unit, all housed within a housing 23 that can be attached to the wearable device 3 using a hook 27. Additionally, the housing 23 features a slot for a plug connection 25, enabling the connection of a wire 21 from the sensor 13 to the control unit 19 and/or for charging the battery. In an alternative embodiment, the control unit 19 can be directly embedded into the elastomer support 5.

The wearable device 3 includes an opening 15 that matches the groove 7 of the elastomer support 5 in the elastomer support assembly 1. Accordingly, the width of the groove 7 substantially corresponds to the thickness of the wearable device 3. Since the elastomer support 5 is flexible, it can be easily threaded through the opening 15, allowing the wearable device 3 or belt to suspend and be positioned within the groove 7 of the elastomer support 5. The edge areas 29 of the opening 15 are reinforced with silicone for added durability.

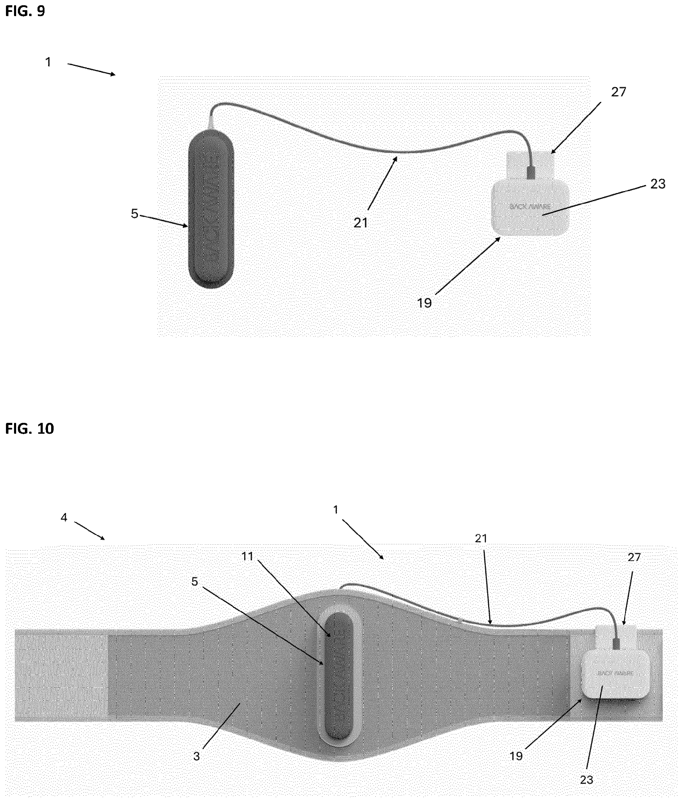

**Fig. 8** and **Fig.9** show a preferred embodiment of the elastomer support assembly in an assembled view and a disassembled view, respectively. Reference is made to the descriptions of the other figures.

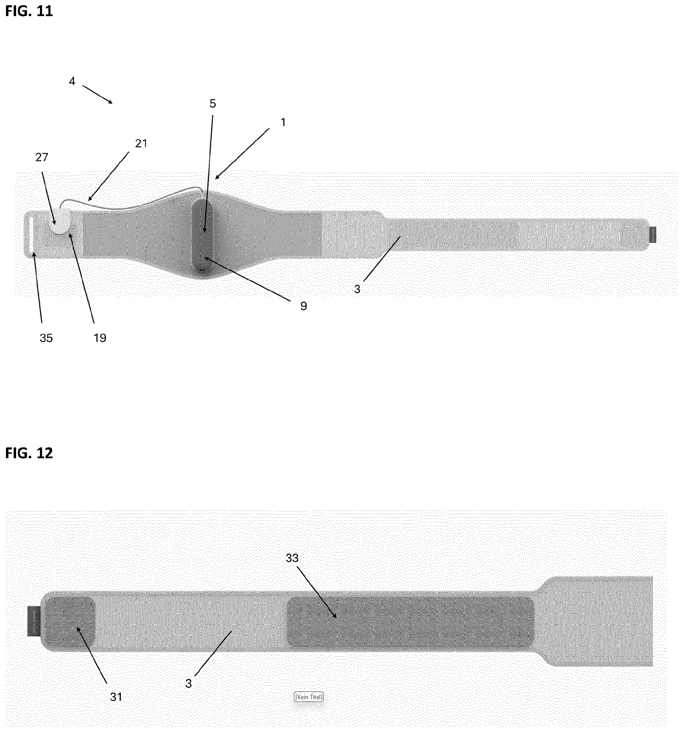

**Fig. 10****,** **Fig. 11** and **Fig. 12** show partial views of a preferred embodiment of the back posture monitoring system 4 from behind, focusing particularly on the central section and each outer section of the wearable device 3. Reference is made to the descriptions of the other figures.

The wearable device 3 is in the form of a belt. The belt comprises an elongate strap on one outer section. The strap is preferably configured with Velcro patches 31, 33 through which the user 37 is able to adjust the size and position of the belt, thereby ensuring a precise fit for every body type. Additionally, different lengths of this part of the belt can be implemented to accommodate various percentiles.

The other outer section of the belt include a small-sized strap having a closure opening 35 formed with a frame similar to the opening 15 in the central section in the wearable device 3. The closure opening 35 is configured to receive the strap which is adjustable using the Velcro patches 31,33.

The frame of the closure opening is made of silicone that prevents the fabric from tearing while also ensuring that the strap slides properly when the belt is adjusted. The straps can be made of medium-elasticity fiber while the center section of the belt is formed of thick stretch spandex. This fabric allows increased adjustability to the body while helps adjusting the elastomer support holding the sensor.

The frames of the openings 15, 35 are preferably formed of co-injection molding, which method involves co-injecting both fabric and silicone to create a seamless integration. The fabric provides strength and flexibility, while the silicone ensures a secure and comfortable fit. This technique offers durability and a clean, finished appearance. In another embodiment, the frames of the openings 15, 35 are formed of plastic. The plastic frames are sewn, snapped, or heat-stamped into place. These plastic frames provide a rigid structure that supports the silicone, while ensuring it maintains its shape and function over time. The choice of plastic material and method of attachment are adjustable to meet durability and cost requirements.

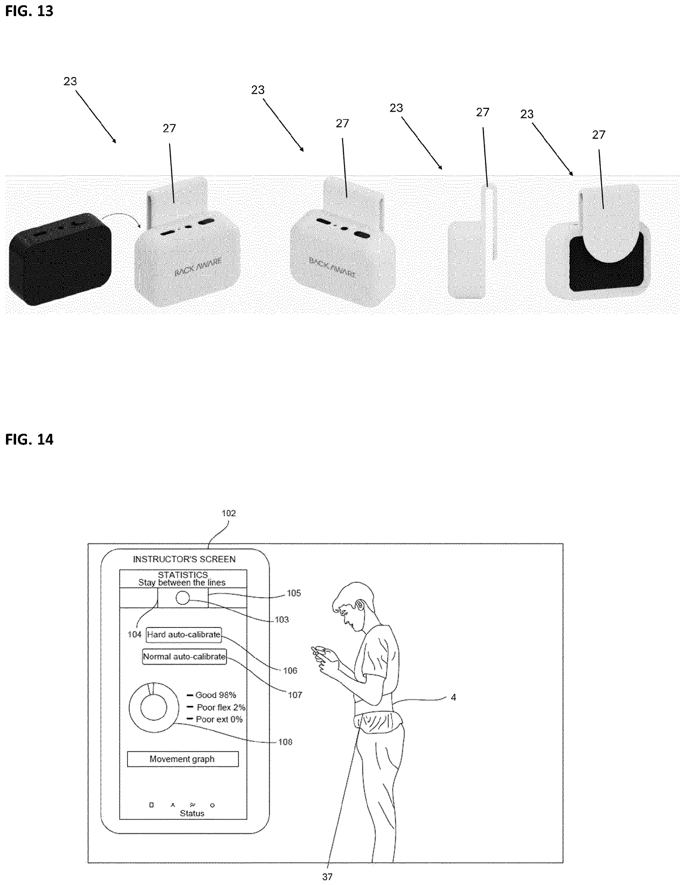

**Fig. 13** shows different views of a housing 23 of the control unit 19 in an elastomer support assembly 1. The housing 23 is at least partially encased in a silicone cover. Instead of the housing 23 itself having a hook 27, the hook 27 can alternatively be provided solely through the silicone cover. Multiple connectors are included to allow devices other than sensors to be connected to the control unit 19. For example, a charger or headphones could be connected if the control unit 19 also includes a memory and means for playing music or similar media.

**Fig. 14** shows a user 37 standing straight, wearing a belt integrated with elastomer support assembly 1, and a screen of the user's smart device 102 is shown.

The posture monitoring system 4 may include an initial calibration process. After the user 37 wears the system 4, they can calibrate it using a mobile or computer application. This calibration ensures that the sensor 13 detects movements relative to a defined baseline or zero point, eliminating inconsistencies in feedback and enabling the sensor 13 to provide accurate data during the user's activities.

The posture monitoring system 4 is to be dynamic, since the posture monitoring system 4 is capable of monitoring the user's posture in real time, and adapt the ideal spinal position based on the user's desired posture or, alternatively, automatically based on predetermined factors. The posture monitoring system 4 is capable of functioning continuously, regardless of the user's movements or activities. This functionality is preferably achieved through the integration of the posture sensor 13 and the control unit 19, which work in tandem to consistently capture the spinal position and provide immediate feedback as needed.

Since the posture monitoring system 4 is further enhanced by the mobile application which is accessible via the smart device, the posture monitoring system 4 allows for recalibration of the posture sensor 13 in accordance with the user's desired spinal position, or automatically based on predetermined factors. This recalibration feature enables the user 13 to reset the zero value of the sensors at any time, while allowing the setting of a new ideal posture or spinal position based on specific activities, such as exercises.

The user 37 is allowed to visualize the back posture via the GUI on the smart device which displays back posture through a digital sensor ball 107 and digital flexion 108 and extension barriers 109. The flexion 108 and extension barriers 109 are upper and lower limits respectively alerting the user of excessive movement. The flexion 108 and extension barriers 109 return to predetermined distances from the desired position upon recalibration.

### REFERENCE NUMERAL LIST

- 1: elastomer support assembly
- 3: wearable device
- 4: system to monitor back posture
- 5: elastomer support
- 7: groove
- 9: first section support section
- 11: second section support section
- 13: sensor
- 15: opening in wearable device
- 17: cavity
- 19: control unit
- 21: wire
- 23: housing
- 25: plug
- 27: hook
- 29: reinforcement material
- 31: velcro patch
- 33: velcro patch
- 35: closure opening
- 37: user
- 102: screen of user's smart device
- 103: sensor ball
- 104: flexion line (upper limit)
- 105: extension line (lower limit)
- 108: movement graph

## Claims

1. An elastomer support assembly (1) for use in connection with a wearable device (3) to monitor back posture, in particular real time spinal position, comprising:
a. an elastomer support (5);
b. a sensor (13) detachably integrated within the elastomer support (5);
wherein the elastomer support (5) is attachable to the wearable device (3) and adhering directly to the lumbar spine of a user (37) when the wearable device (3) is worn by the user (37) for allowing sensors (13) to detect lower back movement of the user (37).

2. The elastomer support (1) assembly according to claim 1,
wherein the elastomer support (5) comprises a circumferential groove (7), which divides the elastomer support (5) into a first and second support section (9, 11);
wherein the elastomer support (5) is attachable to the wearable device (3) through an opening (15) that matches the groove (7), positioning the first support section (9) on an inner side and the second support section (11) on an outer side of the wearable device (3) with the first support section (9) adhering to the lumbar spine of a user when the wearable device (3) is worn by the user (37).

3. The elastomer support (1) assembly according to claim 2,
wherein the outer contour of the second support section (11) has smaller dimensions than that of the first section (9).

4. The elastomer support assembly (1) according to claim 2 or claim 3, wherein the first support section (9) comprises a cavity (17) in which the sensor (13) is placed, the cavity (17) being accessible from the outside, allowing the sensor (13) to be connected to a wire (21) and allowing the sensor to be removed from the elastomer support (5).

5. The elastomer support assembly (1) according to any of the preceding claims, further comprising an electronic control unit (19) which is connected to the sensor (13) via a wire (21) to continuously transmit sensor data related to back posture of the user (37) in real-time.

6. The elastomer support assembly (1) according to claim 5, wherein the control unit (19) comprises a processor, a memory, a rechargeable battery and/or a data communication unit housed in a housing (23) attachable via attaching means to the wearable device (3).

7. The elastomer support assembly (1) according to claim 5 or 6, wherein the sensor data includes spine position data of the user (37) and wherein the control unit (19) is configured to detect if the spine position of the user (37) exceeds predefined thresholds.

8. The elastomer support assembly (1) according to any of the preceding claims, wherein the elastomer support (5) is made of flexible materials including foam, rubber and/or silicone.

9. The elastomer support assembly (1) according to any of the preceding claims, wherein the sensor (13) includes a flex sensor, a strain sensor, and any other position and displacement detection sensor.

10. A wearable device (3) for use in connection with an elastomer support assembly (1) according to any of the preceding claims for monitoring back posture, in particular real time spinal position, wherein the wearable device (3) comprises an opening (15) that matches a groove (7) of the elastomer support (5) of the elastomer support assembly (1).

11. The wearable device (3) according to claim 10, comprising a belt, a garment or any other body-wearable apparatus.

12. The wearable device (3) according to claim 10 or claim 11, wherein edge areas of the opening are reinforced either with reinforcement materials (29) or through processing techniques such as heat sealing, stitching, or adhesive bonding.

13. The wearable device (3) according to claim 11, wherein the belt comprises different sections along the length of the belt which differ in material, thickness and/or width.

14. A system (4) to monitor back posture, in particular real time spinal position, comprising:
a. elastomer support assembly (1) according to any of the preceding claims 1-9;
b. a wearable device (3) according to any of the preceding claims 10 - 13;
wherein the elastomer support (5) is attached to the wearable device (3) through the opening (15), positioning the first support section (9) on an inner side and the second support section (11) on an outer side of the wearable device (3) with the first support section (9) adhering to the lumbar spine of a user (37) when the wearable device (3) is worn by the user (37) for allowing sensors (13) to detect lower back movement of the user (37).

15. The system according to claim 14, wherein the elastomer support assembly (1) includes a control unit (19) enclosed in a housing (23) with a hook (27), the control unit being attached to the wearable device (3) via the hook (27) and connected to the sensor of the elastomer support assembly (1) through a wire (21).

## Amended claims

### Amended claims in accordance with Rule 137(2) EPC.

**1.** An elastomer support assembly (1) for use in connection with a wearable device (3) to monitor back posture, in particular real time spinal position, comprising:
a. an elastomer support (5);
b. a sensor (13) detachably integrated within the elastomer support (5);
**characterized in that**
the elastomer support (5) comprises a circumferential groove (7), which divides the elastomer support (5) into a first and second support section (9, 11);
wherein the elastomer support (5) is attachable to the wearable device (3) through an opening (15) that matches the groove (7), positioning the first support section (9) on an inner side and the second support section (11) on an outer side of the wearable device (3) with the first support section (9) adhering directly to the lumbar spine of a user (37) when the wearable device (3) is worn by the user (37) for allowing sensors (13) to detect lower back movement of the user (37).
wherein the elastomer support (5) is flexible enough to conform to different body shapes.

**2.** The elastomer support (1) assembly according to claim 1,
wherein the outer contour of the second support section (11) has smaller dimensions than that of the first section (9).

**3.** The elastomer support assembly (1) according to claim 1 or claim 2, wherein the first support section (9) comprises a cavity (17) in which the sensor (13) is placed, the cavity (17) being accessible from the outside, allowing the sensor (13) to be connected to a wire (21) and allowing the sensor to be removed from the elastomer support (5).

**4.** The elastomer support assembly (1) according to any of the preceding claims, further comprising an electronic control unit (19) which is connected to the sensor (13) via a wire (21) to continuously transmit sensor data related to back posture of the user (37) in real-time.

**5.** The elastomer support assembly (1) according to claim 4, wherein the control unit (19) comprises a processor, a memory, a rechargeable battery and/or a data communication unit housed in a housing (23) attachable via attaching means to the wearable device (3).

**6.** The elastomer support assembly (1) according to claim 4 or 5, wherein the sensor data includes spine position data of the user (37) and wherein the control unit (19) is configured to detect if the spine position of the user (37) exceeds predefined thresholds.

**7.** The elastomer support assembly (1) according to any of the preceding claims, wherein the elastomer support (5) is made of flexible materials including foam, rubber and/or silicone.

**8.** The elastomer support assembly (1) according to any of the preceding claims, wherein the sensor (13) includes a flex sensor, a strain sensor, and any other position and displacement detection sensor.

**9.** A system (4) to monitor back posture, in particular real time spinal position, comprising:
a. elastomer support assembly (1) according to any of the preceding claims 1- 9;
b. a wearable device (3) comprising an opening (15) that matches the groove (7) of the elastomer support (5) of the elastomer support assembly (1);
wherein the elastomer support (5) is attached to the wearable device (3) through the opening (15), positioning the first support section (9) on an inner side and the second support section (11) on an outer side of the wearable device (3) with the first support section (9) adhering to the lumbar spine of a user (37) when the wearable device (3) is worn by the user (37) for allowing sensors (13) to detect lower back movement of the user (37).

**10.** The system according to claim 9, wherein the elastomer support assembly (1) includes a control unit (19) enclosed in a housing (23) with a hook (27), the control unit being attached to the wearable device (3) via the hook (27) and connected to the sensor of the elastomer support assembly (1) through a wire (21).

**11.** The system according to claim 9 or claim 10, wherein the wearable device (3) comprises a belt, a garment or any other body-wearable apparatus.

**12.** The system according to any of the preceding claims 9 - 11, wherein edge areas of the opening (15) are reinforced either with reinforcement materials (29) or through processing techniques such as heat sealing, stitching, or adhesive bonding.

**13.** The system according to claim 11, wherein the belt comprises different sections along the length of the belt which differ in material, thickness and/or width.

**1.** An elastomer support assembly (1) for use in connection with a wearable device (3) to monitor back posture, in particular real time spinal position, comprising:
a. an elastomer support (5);
b. a sensor (13) detachably integrated within the elastomer support (5);
**characterized in that**
the elastomer support (5) comprises a circumferential groove (7), which divides the elastomer support (5) into a first and second support section (9, 11);
wherein the elastomer support (5) is attachable to the wearable device (3) through an opening (15) that matches the groove (7), positioning the first support section (9) on an inner side and the second support section (11) on an outer side of the wearable device (3) with the first support section (9) adhering directly to the lumbar spine of a user (37) when the wearable device (3) is worn by the user (37) for allowing sensors (13) to detect lower back movement of the user (37);
wherein elastomer support (5) comprising a height, a width and a depths, the height of the elastomer support (5) being greater than its width and depth and it is aligned with its height along the spine, wherein the height is large enough to detect the relevant changes in spinal position that occur along a vertical axis when the user moves;
wherein the elastomer support (5) is flexible enough to conform to different body shapes.

**2.** The elastomer support (1) assembly according to claim 1,
wherein the outer contour of the second support section (11) has smaller dimensions than that of the first section (9).

**3.** The elastomer support assembly (1) according to claim 1 or claim 2, wherein the first support section (9) comprises a cavity (17) in which the sensor (13) is placed, the cavity (17) being accessible from the outside, allowing the sensor (13) to be connected to a wire (21) and allowing the sensor to be removed from the elastomer support (5).

**4.** The elastomer support assembly (1) according to any of the preceding claims, further comprising an electronic control unit (19) which is connected to the sensor (13) via a wire (21) to continuously transmit sensor data related to back posture of the user (37) in real-time.

**5.** The elastomer support assembly (1) according to claim 4, wherein the control unit (19) comprises a processor, a memory, a rechargeable battery and/or a data communication unit housed in a housing (23) attachable via attaching means to the wearable device (3).

**6.** The elastomer support assembly (1) according to claim 4 or 5, wherein the sensor data includes spine position data of the user (37) and wherein the control unit (19) is configured to detect if the spine position of the user (37) exceeds predefined thresholds.

**7.** The elastomer support assembly (1) according to any of the preceding claims, wherein the elastomer support (5) is made of flexible materials including foam, rubber and/or silicone.

**8.** The elastomer support assembly (1) according to any of the preceding claims, wherein the sensor (13) includes a flex sensor, a strain sensor, and any other position and displacement detection sensor.

**9.** A system (4) to monitor back posture, in particular real time spinal position, comprising:
a. elastomer support assembly (1) according to any of the preceding claims 1- 9;
b. a wearable device (3) comprising an opening (15) that matches the groove (7) of the elastomer support (5) of the elastomer support assembly (1);
wherein the elastomer support (5) is attached to the wearable device (3) through the opening (15), positioning the first support section (9) on an inner side and the second support section (11) on an outer side of the wearable device (3) with the first support section (9) adhering to the lumbar spine of a user (37) when the wearable device (3) is worn by the user (37) for allowing sensors (13) to detect lower back movement of the user (37).

**10.** The system according to claim 9, wherein the elastomer support assembly (1) includes a control unit (19) enclosed in a housing (23) with a hook (27), the control unit being attached to the wearable device (3) via the hook (27) and connected to the sensor of the elastomer support assembly (1) through a wire (21).

**11.** The system according to claim 9 or claim 10, wherein the wearable device (3) comprises a belt, a garment or any other body-wearable apparatus.

**12.** The system according to any of the preceding claims 9 - 11, wherein edge areas of the opening (15) are reinforced either with reinforcement materials (29) or through processing techniques such as heat sealing, stitching, or adhesive bonding.

**13.** The system according to claim 11, wherein the belt comprises different sections along the length of the belt which differ in material, thickness and/or width.
